Europäisches Patentamt

European Patent Office

Office européen des brevets

(11) EP 0 813 859 A2

(19)

(12) **EUROPÄISCHE PATENTANMELDUNG**

(43) Veröffentlichungstag:
29.12.1997 Patentblatt 1997/52

(51) Int. Cl.⁶: $A61K\ 7/06$

(21) Anmeldenummer: 97109066.7

(22) Anmeldetag: 05.06.1997

(84) Benannte Vertragsstaaten:
AT BE CH DE DK ES FI FR GB GR IE IT LI LU MC NL PT SE

(30) Priorität: 22.06.1996 DE 19625015

(71) Anmelder:
Wella Aktiengesellschaft
64274 Darmstadt (DE)

(72) Erfinder:
• Kripp, Thomas, Dr.
64407 Fränkisch-Crumbach (DE)

• Hölzel, Hans
64407 Fränkisch-Crumbach (DE)
• Krause, Beate
65795 Hattersheim (DE)
• Gollin, Wolfram
64289 Darmstadt (DE)

(74) Vertreter:
KEIL & SCHAAFHAUSEN
Patentanwälte
Eysseneckstrasse 31
60322 Frankfurt am Main (DE)

(54) **Haarbehandlungsmittel mit Depotwirkung**

(57) Gegenstand der vorliegenden Erfindung ist ein emulsionsförmiges Haarbehandlungsmittel, enthaltend

a) 0,5 bis 15 Gewichtsprozent eines Lipids ausgewählt aus natürlichen oder synthetischen $C_{40}$- bis $C_{140}$-Wachsestern, $C_{26}$- bis $C_{100}$-Kohlenwasserstoffen, $C_{26}$- bis $C_{80}$-Fettalkoholen, $C_{22}$- bis $C_{80}$-Fettsäuren oder deren Gemisch und

b) 0,01 bis 5 Gewichtsprozent mindestens eines in der Schmelze der Komponente a) löslichen kosmetischen Wirkstoffs, ausgewählt aus Antischuppenwirkstoffen, Vitaminen, Duftstoffen, desodorierenden Wirkstoffen, Haarwuchsmitteln und Lichtschutzmitteln,

wobei die Komponenten a) und b) in einem Gewichtsverhältnis von b) zu a) von 1:99 bis 60:50 vorliegen.
Durch die Verwendung des erfindungsgemäßen Mittels kann, trotz des Ausspülens des Mittels nach der Einwirkungszeit mit Wasser, in vorteilhafter Weise ein kosmetischer Wirkstoff gemäß Komponente b) auf dem Haar und der Kopfhaut deponiert werden.

**Beschreibung**

Gegenstand der vorliegenden Erfindung ist ein auszuspülendes Haarbehandlungsmittel mit einem Gehalt an einer Kombination aus bestimmten hochschmelzenden, vorzugsweise gesättigten Lipiden und einem kosmetischen Wirkstoff, bei dessen Anwendung nach seinem Ausspülen mit Wasser eine gegenüber üblichen Haarbehandlungsmitteln verstärkte Ablagerung des enthaltenen Wirkstoffs auf Haar und Kopfhaut erfolgt.

Haarbehandlungsmittel lassen sich nach ihrem Anwendungsverfahren in solche, die im Haar verbleiben und jene, die nach einer gewissen Einwirkungszeit mit Wasser ausgespült werden, einteilen.

Bei auszuspülenden Haarbehandlungsmitteln mit pflegender Wirkung ist das Verbleiben eines Teils der pflegenden, glanzfördernden und kämmbarkeitsverbessernden Substanzen auf dem Haar erwünscht.

Um dies zu erreichen, wurden in der Vergangenheit vor allem pflegende, glanzfördernde und kämmbarkeitsverbessernde Substanzen mit freien kationischen Gruppen, wie zum Beispiel kationische Tenside und kationische Polymere, eingesetzt.

Die Fixierung von Wirkstoffen auf dem Haar durch kationische Gruppen hat jedoch eine Reihe von Nachteilen. So kann der kationische Wirkstoff ungleichmäßig auf das Haar aufziehen, da er sich bevorzugt an geschädigte Stellen des Haares mit hoher negativer Ladungsdichte ablagert. Kationische Polymere weisen zudem den Nachteil auf, daß sie das Haar belasten und das Haar infolge in kürzeren Zeitabständen gewaschen werden muß. Auf der Kopfhaut können durch diese kationische Fixierung nur sehr geringe Mengen des Wirkstoffs deponiert werden. Auch auf dem Haar ist die Menge des deponierten Wirkstoffs sehr gering. Zudem ist die Ablagerung kationischer Wirkstoffe auf dem Haar sehr stark pH-abhängig, da das Haar unterhalb von pH 3 eine positive Nettoladung aufweist, so daß kationische Wirkstoffe nicht mehr haften.

Aufgabe der vorliegenden Erfindung ist es daher, ein mit Wasser auszuspülendes kosmetisches Mittel zur Verfügung zu stellen, durch das ein Wirkstoff auf dem Haar und der Kopfhaut deponiert werden kann und das die vorstehend genannten Nachteile vermeidet.

Es wurde nun gefunden, daß ein emulsionsförmiges Haarbehandlungsmittel enthaltend

a) 0,5 bis 15 Gewichtsprozent mindestens eines, vorzugsweise gesättigten, Lipids, ausgewählt aus natürlichen oder synthetischen $C_{40}$- bis $C_{140}$-Wachsestern, $C_{26}$- bis $C_{100}$-Kohlenwasserstoffen, $C_{26}$- bis $C_{80}$-Fettalkoholen, $C_{22}$- bis $C_{80}$-Fettsäuren oder deren Gemisch und

b) 0,01 bis 5 Gewichtsprozent mindestens eines in der Schmelze von Komponente a) löslichen kosmetischen Wirkstoffs, ausgewählt aus Antischuppenwirkstoffen, Vitaminen, Duftstoffen, desodorierenden Wirkstoffen, Haarwuchsmitteln und Lichtschutzmitteln,
wobei die Komponenten a) und b) in einem Gewichtsverhältnis von b) zu a) von 1:99 bis 60:50 vorliegen,

die gestellte Aufgabe in hervorragender Weise löst.

Durch die Verwendung des erfindungsgemäßen Mittels kann trotz Ausspülen des Mittels nach der Einwirkungszeit mit Wasser, in vorteilhafter Weise ein kosmetischer Wirkstoff gemäß Komponente b) auf dem Haar und der Kopfhaut deponiert werden.

Das erfindungsgemäße Mittel enthält bevorzugt 1,0 bis 5 Gewichtsprozent der Komponente a), wobei Paraffin mit einem Schmelzpunkt von 50 bis 90 °Celsius, Vaseline mit einem Schmelzpunkt von mindestens 50 °Celsius, $C_{26}$- bis $C_{30}$-Fettalkohole, besonders bevorzugt Triacontanol, 1-Hexacosanol und 1-Tetracosanol, $C_{22}$- bis $C_{30}$-Fettsäuren, besonders bevorzugt Melissinsäure und Cerotinsäure, Tetracosylmelissat und $C_{20}$- bis $C_{30}$-Wachsester, besonders bevorzugt Melissinylcerotinat, Tetracosylmelissat, Docosanylbehenat und Melissinsäuretriacontylester, Orangenwachs oder Flachswachs, bevorzugt enthalten sind.

Die Komponente b) ist in dem erfindungsgemäßen Mittel, bevorzugt in einer Menge von 0,01 bis 5,0 Gewichtsprozenten, enthalten.

Als Antischuppenwirkstoff enthält die Komponente b) bevorzugt das unter dem Handelsnamen Octopirox® von der Firma Hoechst AG, Frankfurt, BRD vertriebene 1-Hydroxy-4-methyl-6-(2,4,4-trimethylpentyl)-2(1H-pyridon)-Monoethanolaminsalz und/oder das von der Firma Bayer AG, Leverkusen, BRD unter der Handelsbezeichnung Climbazol® vertriebene 1-(4-Chlorphenoxy)-1-(1-imidazoyl)-3,3-dimethyl-2-butanon. Als Vitamine enthält die Komponente b) bevorzugt Biotin oder Vitamin E.

Als Duftstoff enthält die Komponente b) des erfindungsgemäßen Mittels bevorzugt Zedernholzöl, Menthol, Eukalyptusöl, Campher und/oder Parfümöle.

Als desodorierenden Wirkstoff enthält der Komponent b) bevorzugt Ursolsäure, Oleanolsäure oder desodorierend wirkende ätherische Öle, wie zum Beispiel Teebaumöl (Melalenka alterni folium Öl).

Als Haarwinchsmittel enthält die Komponente b) bevorzugt Biotin.

Als Lichtschutzfilter enthält die Komponente b) des erfindungsgemäßen Mittels bevorzugt Benzophenon und/oder Derivate des Benzophenons, Salicylsäure- und Zimtsäureester, wie zum Beispiel das unter der Handelsbezeichung Neo-Heliopan® BB von der Firma Harmann & Reimer, Holzminden, BRD vertriebene 2-Hydroxy-4-methoxybenzophenon oder der unter der Handelsbezeichung Neo-Heliopan® AV vertriebene p-Methoxyzimtsäure-2-äthylhexylester.

Das erfindungsgemäße Mittel enthält die Komponenten b) und a) vorzugsweise in einem Gewichtsverhältnis von b) zu a) von 20:80 bis 50:50.

Das erfindungsgemäße Haarbehandlungsmittel weist bevorzugt einen Wassergehalt von 10 bis 98 Gewichtsprozent und einen pH-Wert von 2 bis 7 auf, wobei zur Einstellung des pH-Wertes physiologisch verträgliche Säuren, beispielsweise Zitronensäure, Milchsäure, Ameisensäure oder Glyoxylsäure, verwendet werden.

Das erfindungsgemäße Haarbehandlungsmittel kann als Haarpflegemittel, beispielsweise als Haarspülung oder Haarkur sowie als Haarfestiger oder als Haarreinigungsmittel vorliegen.

Ein erfindungsgemäßes Haarreinigungsmittel enthält zusätzlich zur Kombination der Komponenten a) und b) 8 bis 20 Gewichtsprozent, bevorzugt 10 bis 15 Gewichtsprozent, anionische, kationische, amphotere und/oder nicht-ionische Tenside.

Eine bevorzugte Ausführungsform des erfindungsgemäßen Mittels ist ein Antischuppen-Haarreinigungsmittel, eine Antischuppen-Haarspülung oder eine Antischuppen-Haarkur, welche als Komponente b) 1-Hydroxy-4-methyl-6-(2,2,4-trimethylpentyl)-2(1H-pyridon)-Monoethanolaminsalz und zusätzlich als Komponente c)

0,1 bis 10 Gewichtsprozent, bevorzugt 0,25 bis 6 Gewichtsprozent, besonders bevorzugt 0,25 bis 3 Gewichtsprozent, mindestens eines kationischen Tensids und/oder kationische Stärke enthält, wobei das Antischuppenshampoo einen zusätzlichen Gehalt an 8 bis 20 Gewichtsprozent, bevorzugt 10 bis 15 Gewichtsprozent, anionische, kationische, amphotere und/oder nicht-ionische Tenside aufweist.

Das erfindungsgemäße Mittel gemäß der vorstehenden bevorzugten Ausführungsform enthält bevorzugt kationische Stärke, welche beispielsweise als kationische Kartoffelstärke von der Firma Südstärke GmbH, Schrobenhausen, BRD unter der Handelsbezeichnung Catsol® 12 SSG vertrieben wird.

Ein erfindungsgemäßes Haarbehandlungsmittel kann zusätzlich alle diejenigen Bestandteile enthalten, die in Haarbehandlungsmitteln üblicherweise eingesetzt werden, insbesondere anionische, amphotere, kationische oder nicht-ionische Tenside, Emulgatoren, Schaumsynergisten; Schaumstabilisatoren; Sequestriermittel, Naturstoffe; Pigmente; Parfümöle in einer Menge von 0,1 bis 5,0 Gewichtsprozent; Trübungsmittel, wie zum Beispiel Ethylenglykoldistearat, in einer Menge von etwa 0,2 bis 5,0 Gewichtsprozent; Perlglanzmittel-Dispersionen, wie zum Beispiel ein Gemisch aus Fettsäuremonoalkylolamid und Ethylenglykoldistearat, in einer Menge von etwa 1,0 bis 10,0 Gewichtsprozent; Verdickungsmittel, wie beispielsweise Kokosfettsäurediethanolamid oder Hydroxyalkylcellulose, in einer Menge von 0,1 bis 10,0 Gewichtsprozent; Puffersubstanzen, wie beispielsweise Natriumcitrat oder Natriumphosphat in einer Menge von 0,1 bis 1,0 Gewichtsprozent; sowie Haar- und Produktfärbestoffe, wie zum Beispiel Fluorescein-Natriumsalz, Gelb ZN 3 (C. I. 47055), in einer Menge von 0,01 bis 1,0 Gewichtsprozent; weiterhin haarpflegende Zusätze wie zum Beispiel Aminosäuren, Proteine, Kräuterextrakte, Betaine, Vitamine, Kohlenhydrate und -derivate, Harnstoff, ätherische Öle, Fettsäureester, Fettalkohole, Fettsäureglyceride, ethoxylierte oder propoxylierte gesättigte Fettalkohole; natürliche, modifizierte natürliche oder synthetische Polymere, wie zum Beispiel Schellack oder Polyvinylpyrrolidon/Vinylacetat-Copolymer, kationische, anionische oder nicht-ionische Cellulosederivate, Chitosan, kationische Chitin- oder Chitosanderivate oder Polymerisate von Acrylsäurederivaten; Pflegestoffe, wie zum Beispiel Lanolinderivate, Cholesterin und Pantothensäure, in einer Menge von 0,1 bis 10 Gewichtsprozent; außerdem physiologisch verträgliche anorganische Salze, wie zum Beispiel Natriumchlorid und Natriumsulfat; sowie ferner Feuchthaltemittel; Lichtschutzmittel; Antioxidantien; Komplexbildner; pflegende Lipide mineralischen oder biogenen Ursprungs, wie zum Beispiel kosmetische Öle, Fette, Terpene, Steroide und Wachse sowie Konservierungsstoffe, wie zum Beispiel ein Gemisch aus 80 % Phenoxyethanol und 20 % Methyldibromoglutaronitril, das von der Firma Schülke & Mayr unter der Handelsbezeichnung Euxyl® K 400 vertrieben wird; soweit solche Zusätze nützlich und zweckmäßig erscheinen und mit den Bestandteilen des Mittels verträglich sind.

Das erfindungsgemäße Mittel liegt bevorzugt in Form einer Öl-in-Wasser-Emulsion und kann unter Verwendung eines Treibmittels oder mit Hilfe einer mechanisch betriebenen Sprühvorrichtung versprüht werden.

Wenn das erfindungsgemäße Mittel mit Hilfe eines Treibmittels versprüht wird, so enthält es bevorzugt 3 bis 75 Gewichtsprozent des Treibmittels und wird in einem Druckbehälter abgefüllt.

Als Treibmittel sind beispielsweise niedere Alkane, wie zum Beispiel n-Butan, i-Butan und Propan, oder auch deren Gemische mit Dimethylether sowie ferner bei den in Betracht kommenden Drücken gasförmig vorliegende Treibmittel, wie beispielsweise $N_2$, $N_2O$ und $CO_2$ sowie Gemische der vorstehend genannten Treibmittel geeignet.

Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in den das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Ein Haarpflegemittel gemäß der vorliegenden Erfindung wird, üblicherweise nach der Haarwäsche, im handtuchtrockenen Haar je nach Haarfülle in einer Menge von 5 bis 20 g verteilt. Nach einer Einwirkungszeit von 1 bis 15 Minuten wird das Haar mit Wasser ausgespült und sodann getrocknet.

Die Einwirkungszeit des erfindungsgemäßen Mittels hängt innerhalb der gegebenen zeitlichen Grenzen von dem Verwendungszweck des Mittels ab. Handelt es sich um eine Spülung, so beläßt man sie bei 1 bis 5 Minuten auf dem Haar, während bei einer Haarkur eine Einwirkungsdauer von 3 bis 15 Minuten üblich ist.

Ein erfindungsgemäßes Haarreinigungsmittel wird - je nach Haarfülle - in einer Menge von 2 bis 15 g auf den nassen Haar verteilt. Das Haar wird sodann shampooniert, wobei die Einwirkungszeit des Mittels zwischen 10 Sekunden und 15 Minuten dauert, und sodann wird das Haarreinigungsmittel mit Wasser ausgespült.

Die Herstellung der erfindungsgemäßen Mittel erfolgt, soweit nichts anderes beschrieben ist, durch das Lösen der Komponente b) in der geschmolzenen Komponente a) des erfindungsgemäßen Mittels und anschließende Emulgierung der erhaltenen Schmelze in konventioneller Weise in die wäßrige Phase, in welche gegebenenfalls für Haarbehandlungsmittel übliche Zusatzbestandteile eingearbeitet sind.

Die nachstehenden Beispiele sollen den Gegenstand der Erfindung näher erläutern.

Beispiele

### Beispiel 1: Antischuppenshampoo mit Depotfunktion

| | |
|---|---|
| 21,60 g | eines Gemischs aus Natriumlaurylsulfat, Natriumcetylsulfat und Polyoxyethylen-(10)laurylether (Texapon® CS der Firma Henkel KGaA) |
| 5,00 g | Stearinsäure |
| 4,00 g | Behensäure |
| 1,00 g | 1-Hydroxy-4-methyl-6-(2,4,4-trimethylpentyl)-2(1H-pyridon)-Monoethanolaminsalz (Octopyrox® der Firma Hoechst AG, Frankfurt) |
| 0,10 g | eines Gemischs aus Phenoxyethanol und Methyldibromoglutaronitril (Euxyl® K 400 der Firma Schülke & Mayr) |
| 4,00 g | Triethanolamin |
| 3,00 g | Natriumchlorid |
| 61,30 g | Wasser |
| 100,00 g | |

### Beispiel 2: Antischuppenshampoo mit Depotwirkung

| | |
|---|---|
| 21,60 g | eines Gemischs aus Natriumlaurylsulfat, Natriumcetylsulfat und Polyoxyethylen-(10)laurylether (Texapon® CS der Firma Henkel KGaA) |
| 5,00 g | Stearinsäure |
| 4,00 g | Docosanylbehenat |
| 1,00 g | 1-Hydroxy-4-methyl-6-(2,4,4-trimethylpentyl)-2(1H-pyridon)-Monoethanolaminsalz (Octopyrox® der Firma Hoechst AG, Frankfurt) |
| 0,10 g | eines Gemischs aus Phenoxyethanol und Methyldibromoglutaronitril (Euxyl K 400® der Firma Schülke & Mayr) |
| 4,00 g | Triethanolamin |
| 3,00 g | Natriumchlorid |
| 61,30 g | Wasser |
| 100,00 g | |

### Beispiel 3: Antischuppenshampoo mit Depotwirkung

| | |
|---|---|
| 21,60 g | eines Gemischs aus Natriumlaurylsulfat, Natriumcetylsulfat und Polyoxyethylen-(10)laurylether (Texapon® CS der Firma Henkel KGaA) |
| 5,00 g | Stearinsäure |
| 4,00 g | Paraffin mit einem Schmelzpunkt von 87 bis 90 °Celsius |
| 1,00 g | 1-Hydroxy-4-methyl-6-(2,4,4-trimethylpentyl)-2(1H-pyridon)-Monoethanolaminsalz (Octopyrox® der Firma Hoechst AG, Frankfurt) |
| 0,10 g | eines Gemischs aus Phenoxyethanol und Methyldibromoglutaronitril (Euxyl K 400® der Firma Schülke & Mayr) |
| 4,00 g | Triethanolamin |
| 3,00 g | Natriumchlorid |
| 61,30 g | Wasser |
| 100,00 g | |

### Beispiel 4: Antischuppenshampoo mit Depotwirkung

| | |
|---|---|
| 21,60 g | eines Gemischs aus Natriumlaurylsulfat, Natriumcetylsulfat und Polyoxyethylen-(10)laurylether(Texapon® CS der Firma Henkel KGaA) |
| 5,00 g | Stearinsäure |
| 4,00 g | Triacontanol |
| 1,00 g | 1-Hydroxy-4-methyl-6-(2,4,4-trimethylpentyl)-2(1H-pyridon)-Monoethanolaminsalz (Octopyrox® der Firma Hoechst AG, Frankfurt) |
| 0,10 g | eines Gemischs aus Phenoxyethanol und Methyldibromoglutaronitril (Euxyl K 400® der Firma Schülke & Mayr) |
| 4,00 g | Triethanolamin |
| 3,00 g | Natriumchlorid |
| 61,30 g | Wasser |
| 100,00 g | |

**Beispiel 5: Zwei-Phasen-Antischuppenshampoo mit Depotwirkung**

| | |
|---|---|
| 10,80 g | eines Gemischs aus den Natriumsalzen der $C_{12}$- bis $C_{14}$-Alkylglykoläthersulfate |
| 8,00 g | Magnesiumsalz des Polyoxyethylen-3-Kokosfettsäureamidosulfats |
| 0,80 g | 1-Hydroxy-4-methyl-6-(2,4,4-trimethylpentyl)-2(1H-pyridon)-Monoethanolaminsalz (Octopyrox® der Firma Bayer, Leverkusen) |
| 2,00 g | Triglykoldistearat |
| 1,00 g | 1-Hexacosanol |
| 0,50 g | 1-(4-Chlorphenoxy)-1-(1-imidazoyl)-3,3-dimethyl-2-butanon(Climbazol® der Firma Bayer AG, Leverkusen) |
| 0,02 g | eines Gemischs aus Phenoxyethanol und Methyldibromoglutaronitril (Euxyl K 400® der Firma Schülke & Mayr) |
| 4,10 g | Natriumchlorid |
| 72,78 g | Wasser |
| 100,00 g | |

Der pH-Wert des Zwei-Phasen-Antischuppenshampoos wurde mit Zitronensäure auf pH = 6 eingestellt.

Beispiel 6: Zwei-Phasen-Antischuppenshampoo mit **Depotwirkung**

| | |
|---|---|
| 10,80 g | eines Gemischs aus den Natriumsalzen der $C_{12}$- bis $C_{14}$-Alkylglykoläthersulfate |
| 8,00 g | Magnesiumsalz des Polyoxyethylen-3-Kokosfettsäureamidosulfats |
| 0,80 g | 1-Hydroxy-4-methyl-6-(2,4,4-trimethylpentyl)-2(1H-pyridon)-Monoethanolaminsalz (Octopyrox® der Firma Bayer AG, Leverkusen) |
| 2,00 g | Triglykoldistearat |
| 1,00 g | Tetracontan |
| 0,50 g | 1-(4-Chlorphenoxy)-1-(1-imidazoyl)-3,3-dimethyl-2-butanon(Climbazol® der Firma Bayer AG, Leverkusen) |
| 0,02 g | eines Gemischs aus Phenoxyethanol und Methyldibromoglutaronitril (Euxyl K 400® der Firma Schülke & Mayr) |
| 4,10 g | Natriumchlorid |
| 72,78 g | Wasser |
| 100,00 g | |

Der pH-Wert des Zwei-Phasen-Antischuppenshampoos wurde mit Zitronensäure auf pH = 6 eingestellt.

**Beispiel 7: Zwei-Phasen-Antischuppenshampoo mit Depotwirkung**

| | |
|---|---|
| 10,80 g | eines Gemischs aus den Natriumsalzen der $C_{12}$- bis $C_{14}$-Alkylglykoläthersulfate |
| 8,00 g | Magnesiumsalz des Polyoxyethylen-3-Kokosfettsäureamidosulfats |
| 0,80 g | 1-Hydroxy-4-methyl-6-(2,4,4-trimethylpentyl)-2(1H-pyridon)-Monoethanolaminsalz (Octopyrox® der Firma Bayer AG, Leverkusen) |
| 2,00 g | Triglykoldistearat |
| 1,00 g | Vaseline |
| 0,50 g | 1-(4-Chlorphenoxy)-1-(1-imidazoyl)-3,3-dimethyl-2-butanon(Climbazol® der Firma Bayer AG, Leverkusen) |
| 0,02 g | eines Gemischs aus Phenoxyethanol und Methyldibromoglutaronitril (Euxyl K 400® der Firma Schülke & Mayr) |
| 4,10 g | Natriumchlorid |
| 72,78 g | Wasser |
| 100,00 g | |

Der pH-Wert des Zwei-Phasen-Antischuppenshampoos wurde mit Zitronensäure auf pH = 6 eingestellt.

**Beispiel 8: Zwei-Phasen-Antischuppenshampoo mit Depotwirkung**

| | |
|---|---|
| 10,80 g | eines Gemischs aus den Natriumsalzen der $C_{12}$- bis $C_{14}$-Alkylglykoläthersulfate |
| 8,00 g | Magnesiumsalz des Polyoxyethylen-3-Kokosfettsäureamidosulfats |
| 0,80 g | 1-Hydroxy-4-methyl-6-(2,4,4-trimethylpentyl)-2(1H-pyridon)-Monoethanolaminsalz (Octopyrox® der Firma Bayer AG, Leverkusen) |
| 2,00 g | Triglykoldistearat |
| 1,00 g | Flachswachs |
| 0,50 g | 1-(4-Chlorphenoxy)-1-(1-imidazoyl)-3,3-dimethyl-2-butanon(Climbazol® der Firma Bayer AG, Leverkusen) |
| 0,02 g | eines Gemischs aus Phenoxyethanol und Methyldibromoglutaronitril (Euxyl K 400® der Firma Schülke & Mayr) |
| 4,10 g | Natriumchlorid |
| 72,78 g | Wasser |
| 100,00 g | |

Der pH-Wert des Zwei-Phasen-Antischuppenshampoos wurde mit Zitronensäure auf pH = 6 eingestellt.

**Beispiel 9: Duftshampoo mit Depotfunktion für ätherische Öle**

| | |
|---|---|
| 6,00 g | Natriumlaurylsulfat |
| 3,00 g | Glykoldistearat |
| 2,00 g | Paraffin mit einem Schmelzpunkt von 58 bis 60 °Celsius |
| 0,50 g | Zedernholzöl |
| 0,20 g | Eukalyptusöl |

| | |
|---|---|
| 0,10 g | Menthol |
| 1,00 g | Polyoxyethylen-(120)methylglucosedioleat |
| 0,10 g | eines Gemischs aus Phenoxyethanol und Methyldibromoglutaronitril (Euxyl K 400® der Firma Schülke & Mayr) |
| 2,00 g | Natriumchlorid |
| 85,10 g | Wasser |
| 100,00 g | |

**Beispiel 10: Duftshampoo mit Depotfunktion für ätherische Öle**

| | |
|---|---|
| 6,00 g | Natriumlaurylsulfat |
| 3,00 g | Glykoldistearat |
| 2,00 g | Tetracosylmelissat |
| 0,50 g | Zedernholzöl |
| 0,20 g | Eukalyptusöl |
| 0,10 g | Menthol |
| 1,00 g | Polyoxyethylen-(120)-methylglucosedioleat |
| 0,10 g | eines Gemischs aus Phenoxyethanol und Methyldibromoglutaronitril (Euxyl K 400® der Firma Schülke & Mayr) |
| 2,00 g | Natriumchlorid |
| 85,10 g | Wasser |
| 100,00 g | |

**Beispiel 11: Duftshampoo mit Depotfunktion für ätherische Öle**

| | |
|---|---|
| 6,00 g | Natriumlaurylsulfat |
| 3,00 g | Glykoldistearat |
| 2,00 g | Hexacontan |
| 0,50 g | Zedernholzöl |
| 0,20 g | Eukalyptusöl |
| 0,10 g | Menthol |
| 1,00 g | Polyoxyethylen-(120)-methylglucosedioleat |
| 0,10 g | eines Gemischs aus Phenoxyethanol und Methyldibromoglutaronitril (Euxyl K 400® der Firma Schülke & Mayr) |
| 2,00 g | Natriumchlorid |
| 85,10 g | Wasser |
| 100,00 g | |

**Beispiel 12: Duftshampoo mit Depotfunktion für ätherische Öle**

| | |
|---|---|
| 6,00 g | Natriumlaurylsulfat |
| 3,00 g | Glykoldistearat |
| 2,00 g | Cerotinsäure |
| 0,50 g | Zedernholzöl |
| 0,20 g | Eukalyptusöl |
| 0,10 g | Menthol |
| 1,00 g | Polyoxyethylen-(120)-methylglucosedioleat |
| 0,10 g | eines Gemischs aus Phenoxyethanol und Methyldibromoglutaronitril (Euxyl K 400® |

| | |
|---|---|
| | der Firma Schülke & Mayr) |
| 2,00 g | Natriumchlorid |
| 85,10 g | Wasser |
| 100,00 g | |

**Beispiel 13: Duftshampoo mit Depotfunktion für ätherische Öle**

| | |
|---|---|
| 6,00 g | Natriumlaurylsulfat |
| 3,00 g | Glykoldistearat |
| 2,00 g | Orangenwachs |
| 0,50 g | Zedernholzöl |
| 0,20 g | Eukalyptusöl |
| 0,10 g | Menthol |
| 1,00 g | Polyoxyethylen-(120)-methylglucosedioleat |
| 0,10 g | eines Gemischs aus Phenoxyethanol und Methyldibromoglutaronitril (Euxyl K 400® der Firma Schülke & Mayr) |
| 2,00 g | Natriumchlorid |
| 85,10 g | Wasser |
| 100,00 g | |

**Beispiel 14: Haarspülung mit Lichtschutzfaktor**

| | |
|---|---|
| 1,50 g | Kokosfettsäureamidopropylbetain |
| 1,25 g | Cetyltrimethylammoniumchlorid |
| 5,00 g | Hexacosanol |
| 3,00 g | Paraffin mit einem Schmelzpunkt von 87-90 °Celsius |
| 1,00 g | 2-Hydroxy-4-methoxybenzophenon |
| 1,00 g | Zitronensäure |
| 0,20 g | Parfümöl |
| 87,05 g | Wasser |
| 100,00 g | |

**Beispiel 15: Deo-Intensivhaarkur gegen Kopfgeruch**

| | |
|---|---|
| 5,00 g | Cetylalkohol |
| 0,50 g | Bienenwachs |
| 2,00 g | Hexacosanol |
| 0,50 g | 5-Chlor-2-(2,4-dichlorphenoxy)-2,2,4'-trichlor-2'-hydroxydiphenylether |
| 1,50 g | Kokosfettsäureamidopropylbetain |
| 1,00 g | Zitronensäure |
| 0,20 g | Parfümöl |
| 89,30 g | Wasser |
| 100,00 g | |

**Beispiel 16: Antischuppen-Haarkur mit starkem Depoteffekt**

| | |
|---|---|
| 1,000 g | Polyoxyethylen(4)lauryletherphosphorsäuretriester |
| 1,125 g | Trimethylbehenylammoniumchlorid |
| 0,500 g | 1-Hydroxy-4-methyl-6-(2,4,4-trimethyl-pentyl)-2(1H-pyridon)-Monoethanolamin-salz (Octopyrox® der Firma Hoechst AG, |

Frankfurt)

| | |
|---|---|
| 1,000 g | kationische Kartoffelstärke (Catsol® 12 SSG der Firma Südstärke GmbH, Schrobenhausen) |
| 3,000 g | 1-Tetracosanol |
| 2,000 g | Cetylalkohol |
| 1,000 g | Lanolin |
| 0,500 g | Parfümöl |
| 89,875 g | Wasser |
| 100,000 g | |

Für die Herstellung der Antischuppen-Haarkur gemäß Beispiel 16 wird zunächst eine 5prozentige Lösung des 1-Hydroxy-4-methyl-6-(2,4,4-trimethylpentyl)-2(1H-pyridon)-Monoethanolaminsalzes in Propylenglykol mit einer 5prozentigen Lösung der kationischen Kartoffelstärke im Wasser vermischt. Der anfallende Niederschlag wird abfiltriert, getrocknet und in die Schmelze aus Cetylalkohol, Lanolin und 1-Tetracosanol eingearbeitet. Die erhaltene Schmelze wird in konventioneller Weise in die wäßrige Phase emulgiert.

**Beispiel 17: Antischuppen-Haarkur mit starkem Depoteffekt**

| | |
|---|---|
| 1,000 g | Polyoxyethylen(4)lauryletherphosphorsäuretriester |
| 1,125 g | Trimethylbehenylammoniumchlorid |
| 0,500 g | 1-Hydroxy-4-methyl-6-(2,4,4-trimethylpentyl)-2(1H-pyridon)-Monoethanolaminsalz (Octopyrox® der Firma Hoechst AG, Frankfurt) |
| 1,000 g | kationische Kartoffelstärke (Catsol® 12 SSG der Firma Südstärke GmbH, Schrobenhausen) |
| 3,000 g | Cerotinsäure |
| 2,000 g | Cetylalkohol |
| 1,000 g | Lanolin |
| 0,500 g | Parfümöl |
| 89,875 g | Wasser |
| 100,000 g | |

Die Hestellung des Beispiels 17 erfolgt entsprechend dem bei Beispiel 16 beschriebenen Verfahren.

**Beispiel 18: Antischuppen-Haarkur mit starkem Depoteffekt**

| | |
|---|---|
| 1,000 g | Polyoxyethylen(4)lauryletherphosphorsäuretriester |
| 1,125 g | Trimethylbehenylammoniumchlorid |
| 0,500 g | 1-Hydroxy-4-methyl-6-(2,4,4-trimethylpentyl)-2(1H-pyridon)-Monoethanolaminsalz (Octopyrox® der Firma Hoechst AG, Frankfurt) |
| 1,000 g | kationische Kartoffelstärke (Catsol® 12 SSG der Firma Südstärke GmbH, Schrobenhausen) |

| | |
|---|---|
| 3,000 g | Paraffin mit einem Schmelzpunkt von 87-90 °Celsius |
| 2,000 g | Cetylalkohol |
| 1,000 g | Lanolin |
| 0,500 g | Parfümöl |
| 89,875 g | Wasser |
| 100,000 g | |

Die Herstellung des Beispiels 18 erfolgt entsprechend dem bei Beispiel 16 beschriebenen Verfahren.

**Beispiel 19: Antischuppen-Haarkur mit starkem Depoteffekt**

| | |
|---|---|
| 1,000 g | Polyoxyethylen(4)lauryletherphosphorsäuretriester |
| 1,125 g | Trimethylbehenylammoniumchlorid |
| 0,500 g | 1-Hydroxy-4-methyl-6-(2,4,4-trimethylpentyl)-2(1H-pyridon)-Monoethanolaminsalz (Octopyrox® der Firma Hoechst AG, Frankfurt) |
| 1,000 g | kationische Kartoffelstärke (Catsol® 12 SSG der Firma Südstärke GmbH, Schrobenhausen) |
| 3,000 g | Melissinylcerotinat |
| 2,000 g | Cetylalkohol |
| 1,000 g | Lanolin |
| 0,500 g | Parfümöl |
| 89,875 g | Wasser |
| 100,000 g | |

Die Herstellung des Beispiels 19 erfolgt entsprechend dem bei Beispiel 16 beschriebenen Verfahren.

**Patentansprüche**

1. Emulsionsförmiges Haarbehandlungsmittel, enthaltend

   a) 0,5 bis 15 Gewichtsprozent eines Lipids, ausgewählt aus natürlichen oder synthetischen $C_{40}$- bis $C_{140}$-Wachsestern, $C_{26}$- bis $C_{100}$-Kohlenwasserstoffen, $C_{26}$- bis $C_{80}$-Fettalkoholen, $C_{22}$- bis $C_{80}$-Fettsäuren oder deren Gemisch und

   b) 0,01 bis 5 Gewichtsprozent mindestens eines in der Schmelze der Komponente a) löslichen kosmetischen Wirkstoffs, ausgewählt aus Antischuppenwirkstoffen, Vitaminen, Duftstoffen, desodorierenden Wirkstoffen, Haarwuchsmitteln und Lichtschutzmitteln,

   wobei die Komponenten a) und b) in einem Gewichtsverhältnis von b) zu a) von 1:99 bis 60:50 vorliegen.

2. Mittel nach Anspruch 1, dadurch gekennzeichnet, daß die Komponente a) ausgewählt ist aus Paraffin mit einem Schmelzpunkt von 50 bis 90 °Celsius, Vaseline mit einem Schmelzpunkt von mindestens 50 °Celsius, $C_{26}$- bis $C_{30}$-Fettalkoholen, $C_{22}$- bis $C_{30}$-Fettsäuren und $C_{20}$- bis $C_{30}$-Wachsestern.

3. Mittel nach einem der Ansprüche 1 oder 2, dadurch gekennzeichnet, daß die Komponente b) ausgewählt ist aus 1-Hydroxy-4-methyl-6-(2,4,4-trimethylpentyl)-2(1H-pyridon)-Monoethanolaminsalz, 1-(4-Chlorphenoxy)-1-(1-imidazoyl)-3,3-dimethyl-2-butanon, Zedernholzöl, Menthol, Eukalyptusöl, Campher, Parfümöl, Ursolsäure, Oleanolsäure, desodorierend wirkenden ätherischen Ölen, Benzophenon, Derivaten des Benzophenons, Salicylsäure- und/oder Zimtsäureestern.

4. Mittel nach einem der Ansprüche 3, dadurch gekennzeichnet, daß es ein Antischuppen-Haarreinigungsmittel, eine Antischuppen-Haarspülung oder eine Antischuppen-Haarkur ist, welches als Komponente b) 1-Hydroxy-4-methyl-6-(2,2,4-trimethylpentyl)-2(1H-pyridon)-Monoethanolaminsalz und

0,1 bis 10     Gewichtsprozent mindestens eines kationischen Tensids und/oder kationische Stärke

enthält.

5. Mittel nach einem der Ansprüche 1 bis 4, dadurch gekennzeichnet, daß es ein Haarreinigungsmittel ist und 8 bis 20 Gewichtsprozent anionische, kationische amphotere und/oder nicht-ionische Tenside enthält.

6. Mittel nach einem der Ansprüche 1 bis 5, dadurch gekennzeichnet, daß das Gewichtsverhältnis der Komponente b) zur Komponente a) von 20:80 bis 50:50 beträgt.